# NEUE EUROPÄISCHE PATENTSCHRIFT

(11) **EP 1 064 009 B2**
(45) Veröffentlichungstag und Bekanntmachung des Hinweises auf die Entscheidung über den Einspruch: **30.07.2008**
(45) Hinweis auf die Patenterteilung: 02.10.2002
(21) Anmeldenummer: 99913282.2
(22) Anmeldetag: 19.03.1999
(51) Int. Cl.: A61K 36/88, A61K 36/71, A61K 31/35, A61P 15/12

(54) **VERWENDUNG VON EXTRAKTEN AUS IRISGEWÄCHSEN UND CIMICIFUGA RACEMOSA UND TECTORIGENIN ALS ÖSTROGENARTIGES ORGANSELEKTIVES ARZNEIMITTEL OHNE UTEROTROPE WIRKUNG**
UTILIZATION OF EXTRACTS FROM IRIS PLANTS, CIMICIFUGA RACEMOSA AND TECTORIGENIN AS AN ESTROGEN-LIKE ORGAN-SELECTIVE MEDICAMENT WITHOUT UTEROTROPIC EFFECTS
UTILISATION D'EXTRAITS DE PLANTS D'IRIS, DE CIMICIFUGA RACEMOSA ET DE TECTORIGENINE COMME MEDICAMENT OESTROGENOIDE ORGANOSELECTIF SANS EFFET UTEROTROPE

(30) Priorität: 19.03.1998 DE 19812204
(43) Veröffentlichungstag der Anmeldung: 03.01.2001
(73) Patentinhaber: Bionorica AG, 92318 Neumarkt (DE)
(72) Erfinder: WUTTKE, Wolfgang, D-37120 Bovenden (DE); JARRY, Hubertus, D-37249 Neu-Eichenberg (DE); CHRISTOFFEL, Volker, D-92318 Neumarkt (DE); SPENGLER, Barbara, D-92318 Neumarkt (DE); POPP, Michael, D-91207 Lauf (DE)
(74) Vertreter: Kaiser, Jürgen
(86) Internationale Anmeldenummer: PCT/EP1999/001860
(87) Internationale Veröffentlichungsnummer: WO 1999/047149

(56) Entgegenhaltungen:
- DE-C- 19 652 183
- PATENT ABSTRACTS OF JAPAN vol. 012, no. 237 (C-509), 6. Juli 1988 & JP 63 030417 A (TSUMURA JUNTENDO INC), 9. Februar 1988
- American Journal of Natural Medicine, Bd. 4, No. 3, 1997, S. 3-5

## Beschreibung

Die vorliegende Erfindung betrifft die Verwendung von Extrakten aus Irisgewächsen (Iridaceae) gemäß Anspruch 1 sowie aus Cimicifuga racemosa gemäß Anspruch 3 und von Tectorigenin und/oder Tectorigeninglykosiden gemäß Anspruch 5 sowie von Tectorigenin- und/oder Tectorigeninglykosid-haltigen/-angereicheten Extrakten gemäß Anspruch 4 zur Herstellung eines östrogenartigen organselektiven Arzneimittels ohne oder wenigstens mit zu vernachlässigender uterotroper Wirkung.

Das in den Ovarien gebildete 17β-Estradiol (wenn im folgenden von Estradiol gesprochen wird, ist immer das physiologische 17β-Estradiol gemeint) [im folgenden auch mit E₂ bezeichnet] hat im Organismus eine allgemein proliferationsfördernde Wirkung. Neben der Steuerung des Zyklus der Frau besitzt es u.a. einen homöostatischen Einfluss auf den Stoffwechsel des Knochens und verhindert am Endothel der Gefäße die Entstehung atherotischer Plaques.

In der Menopause kommt es zu einem Absinken der Estradiolspiegel in Folge des Erlöschens der Ovarialfunktion. Dies resultiert in einer Abschwächung proliferativer Prozesse und führt im Hypothalamus zu einer Verstärkung der Aktivität des GnRH-Pulsgenerators. (Der Gonadotropin-Releasing-Hormon-Pulsgenerator ist eine Art Taktgeber im Hypothalamus und taktet die pulsatile Ausschüttung von LH, wobei Steroide die Amplitude und die Frequenz beeinflussen.) Die resultierende stimulierte LH-Ausschüttung führt bei der klimakterischen Frau zu störend empfundenen aufsteigenden Hitzwallungen, den sogenannten "Hot flushes".

In Abwesenheit genügend hoher Estradiolspiegel im Blut überwiegt im Knochengewebe die Aktivität der Osteoklasten und damit der Abbau der Knochenmasse, der mit erhöhter Bruchgefahr des Skelettes einhergeht. Gleichzeitig besteht langfristig die Gefahr der Plaquebildung im Gefäßsystem und damit das erhöhte Risiko von Infarkten.

Sowohl von Extrakten aus Cimicifuga racemosa als auch aus Belamcanda chinensis ist aus der Volksmedizin bekannt, dass sie peri- und postmenopausale Beschwerden lindern können. Dies wurde bislang darauf zurückgeführt, dass die Extrakte beider pflanzlicher Drogen estrogenartige Wirkung mit allen deren positiven Wirkungen auf eine Vielzahl von Organen des menschlichen Körpers, insbesondere Gehirn, Ovarien, Knochen, Gefäßsystem, aufweisen. Nachteilig wären estrogenartige Wirkungen auf Uterus, Vagina, Brustgewebe und Leber. Unerwünscht hieran ist jedoch, dass bislang kein Arzneimittel aus diesen Pflanzendrogen im Stand der Technik zur Verfügung stand, welches zu einer organselektiven Prophylaxe oder Therapie bei Estrogenmangel verwendet werden kann.

Ausgehend von diesem Stand der Technik ist es daher Aufgabe der vorliegenden Erfindung, pflanzliche Arzneimittel mit estrogenartiger Wirkung zur Verfügung zu stellen, welche eine organselektive Wirkung ohne oder mit nur geringer Wirkung auf den Uterus aufweisen.

Die Lösung dieser Aufgabe erfolgt unabhängig voneinander durch die Merkmale des Anspruchs 1 bezüglich der Verwendung von Extrakten aus Irisgewächsen (Iridaceae), durch die Verwendung von Extrakten aus Cimicifuga racemosa gemäß Anspruch 3. Die obige Aufgabe wird ferner durch die Merkmale des Anspruchs 2, bezüglich eines Arzneimittels auf Basis von Tectorigenin und/oder dessen Glykosiden gemäß Anspruch 5 gelöst.

Eine weitere unabhängige Lösung stellt ein Tectorigenin und/oder Tectorigeninglykosid-haltiger oder Tectorigenin und/oder Tectorigeninglykosid-angereicherter Pflanzenextrakt gemäß Anspruch 4 dar.

Bei in vitro und in vivo Versuchen hat sich überraschend herausgestellt, dass sowohl aus Irisgewächsen (Iridaceae), insbesondere Belamcanda chinensis, als auch aus Cimifuga racemosa mit organischen Lösungsmitteln oder mit überkritischem CO₂ hergestellte Extrakte organselektiv auf das Zentralnervensystem, das Knochensystem und auf das Gefäßsystem wirken, wobei eine Wirkung auf den Uterus die sogenannte uterotrope Wirkung - fehlt. Daher sind die erfindungsgemäß verwendeten Extrakte gemäß Anspruch 1+4 zur Herstellung eines fertig formulierten Mittels zur selektiven Behandlung und/oder Prophylaxe der Osteoporose und zur selektiven Behandlung und/oder Prophylaxe von kardiovaskulären Erkrankungen, insbesondere der Atherosklerose geeignet.
Die erfindungsgemäßen Extrakte gemäß Anspruch 3 sind zur Herstellung eines fertig formulierten Mittels zur selektiven Behandlung und/oder Prophylaxe von Atherosklerose geeignet.
Es wurde weiterhin gefunden, dass der Inhaltsstoff Tectorigenin, der aus der Belamcanda chinensis isoliert wurde, im wesentlichen die gleichen Wirkungen ausübt wie der Gesamtextrakt.

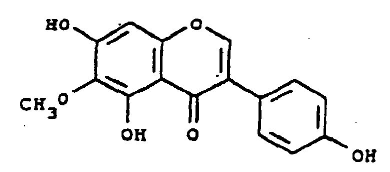

### Tectorigenin

Dieser Inhaltsstoff wird neben der Belamcanda chinensis auch in weiteren Irisgewächsen gefunden, wie z.B. Iris germanica, I. tectorum, I. illyrica, I. dichotoma.

Taxonomisch gesehen stellt sich die Einordnung von Belamcanda chinensis wie folgt dar:

| | |
|---|---|
| Ordnung: | Liliales |
| Familie | Iridaceae |
| Gattung | Belamcanda |
| Art Belameanda chinensis (Leman) DC. = Pardanthus chinensis (L.) | |
| Ker-Gawler auch Ixia chinensis L. (=Gemmingia chinensis (L.) O. Kuntze) | |

Bevorzugt werden zur Herstellung der Extrakte Rhizome, Stengel, Blätter und/oder Blütenblätter der Pflanzen verwendet.

Eine grundsätzliche phytochemische Beschreibung von Belamcanda chinensis und ihrer Inhaltsstoffe erfolgte in der Dissertation von Frau A. Nenninger: (LMU München, 1997) mit dem Titel "Phytochemische und pharmakologische Untersuchungen von Belamcanda chinensis, einer Arzneipflanze der TCM und anderer irisarten".

Mit den erfindungsgemäßen Arzneimitteln stehen erstmals Mittel aus Cimicifuga racemosa und Belamcanda chinensis und anderen Irisarten sowie Mittel auf Tectorigenin-Basis zur Verfügung, welche als volle Estrogenrezeptor-Agonisten in Knochen, dem kardiovaskulären System und dem Gehirn fungieren.

Weitere Vorteile und Merkmale der vorliegenden Erfindung ergeben sich anhand der Beschreibung von experimentellen Daten sowie anhand der Zeichnung.

Es zeigt:
- Fig. 1:: Einen Vergleich der organischen und wässrigen Phase von Cimicifuga racemosa. Verdrängungskurve eines repräsentativen Estrogenrezeptcr - Ligandenbindungsassays. Die Konzentration der Ausgangslösung ist 17,66 mg/ml. Es folgen Verdünnungen 1:2, 1:4 etc. bis 1:64,
- Fig. 2:: Serum LH vor und 2 Stunden nach der intravenösen Injektion von Belamcanda sinensis Extrakt, E2 und Vehikel. Der Belamcanda chinensis Extrakt hat eine ähnliche Potenz, die erhöhten Serum LH-Spiegel zu senken wie E2;
- Fig. 3.: Wirkungen von Cimicifuga racemosa und E2 auf Uterusgewichte (Fig. 3a) und LH Spiegel im Blut (Fig. 3b) in ovariektomierten Ratten nach siebentägiger subkutaner Behandlung; (Mittelwerte + SEM, n = 8, * = p < 0.05 vs. Cremophor als Vehikel);
- Fig. 3a): Uterusgewichte;
- Fig. 3b): LH Konzentrationen im Blut;
- Fig 4a): Wirkungen von Cimicifuga racemosa und E2 in ovariektomierten Ratten nach siebentägiger subkutaner Behandlung; (Mittelwerte + SEM, n = 8, * = p < 0.05 vs. Cremophor als Vehikel) auf die Expression der mRNA für E2- Rezeptor α in der präoptischen Region des Hypothalamus;

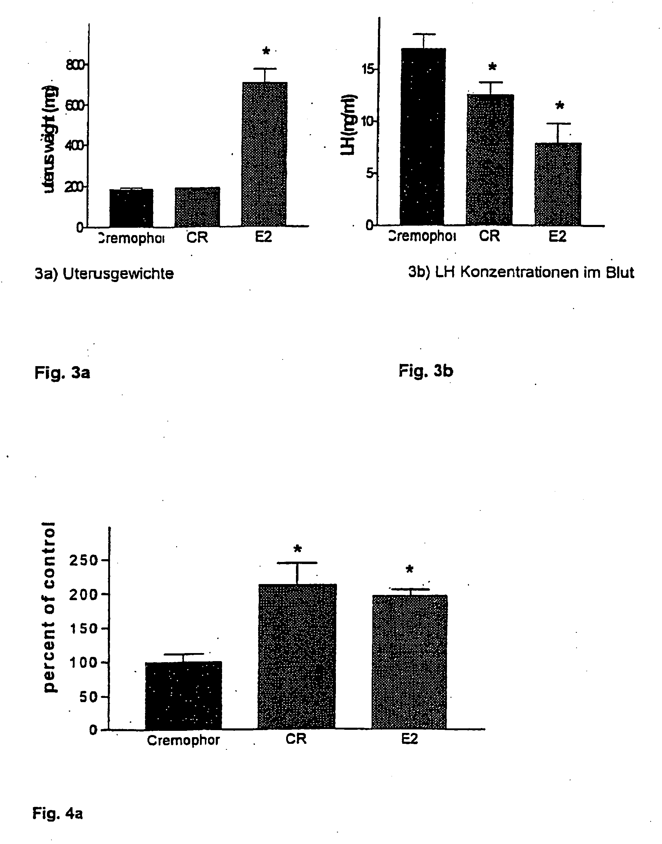

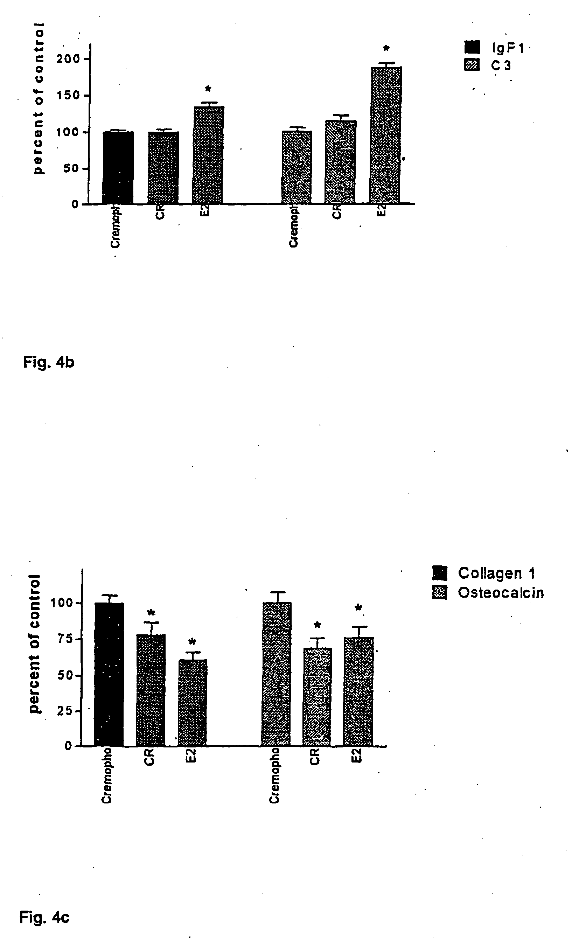

- Fig 4 b): die Expression der mRNA für IGF1 und C3 im Uterus von ovariektomierten Ratten nach 7 Tagen subkutaner Gabe; und
- Fig 4 c): die Expression der mRNA für Collagen 1 (Coll1) und Osteocalcin im Knochen von ovariektomierten Ratten nach 7 Tagen subkutaner Gabe.

### Experimenteller Nachweis der estrogenen Wirkung von Cimicifuga racemosa und Belamcanda chinensis

Der Nachweis der selektiven estrogenen Wirkung wurde stufenweise in einer Reihe von unterschiedlich komplexen Testsystemen geführt.

### 1. in vitro Versuche

### 1.1 in vitro Versuche zu Cimicifuga racemosa

Die Erkennung der estrogenartigen Struktur von Inhaltsstoffen durch einen gegen 17β-Estradiol (=E2) gerichteten Antikörper wurde in vitro gezeigt. Der Cimicifuga racemosa Extrakt wurde zur Trockene gebracht. Durch Phasenverteilung zwischen Dichlormethan und Wasser wurden Substanzen mit unterschiedlicher Polarität angereichert. Die Bindungsaffinität der Inhaltsstoffe der beiden Phasen wurde in vitro an Estrogenrezeptoren aus Schweineuterus bestimmt. Die zytosolischen Estrogenrezeptoren aus den Uteri von Schweinen wurden nach Standardprozeduren isoliert und für die Ligandenverdrängungsexperimente eingesetzt.

Dabei wurde gefunden, daß die estrogenartigen Strukturen z.B. aus Cimicifuga racemosa nicht hydrophil, sondern lipophiler Natur sind, da sie sich mit einem organischen Lösungsmittel aus dem Extrakt extrahieren lassen. Die Substanzen, die sich in der organisch extrahierten Phase befinden, binden etwa zehnfach stärker an den Antikörper als die in der wässrigen Phase verbliebenen Stoffe.

Noch größer ist der Unterschied zwischen den beiden Phasen im Estradiol-Rezeptorbindungsassay. Die Ähnlichkeit der bindenden Substanz mit Estradiol muss so groß sein, dass eine selektive - kompetitive - Interaktion mit dem Estradiolrezeptor in einer zellfreien Präparation stattfinden kann. In diesem Testsystem besitzt die wässrige Phase keine Aktivität, wohingegen die organische Phase sehr fest an den Rezeptor bindet.

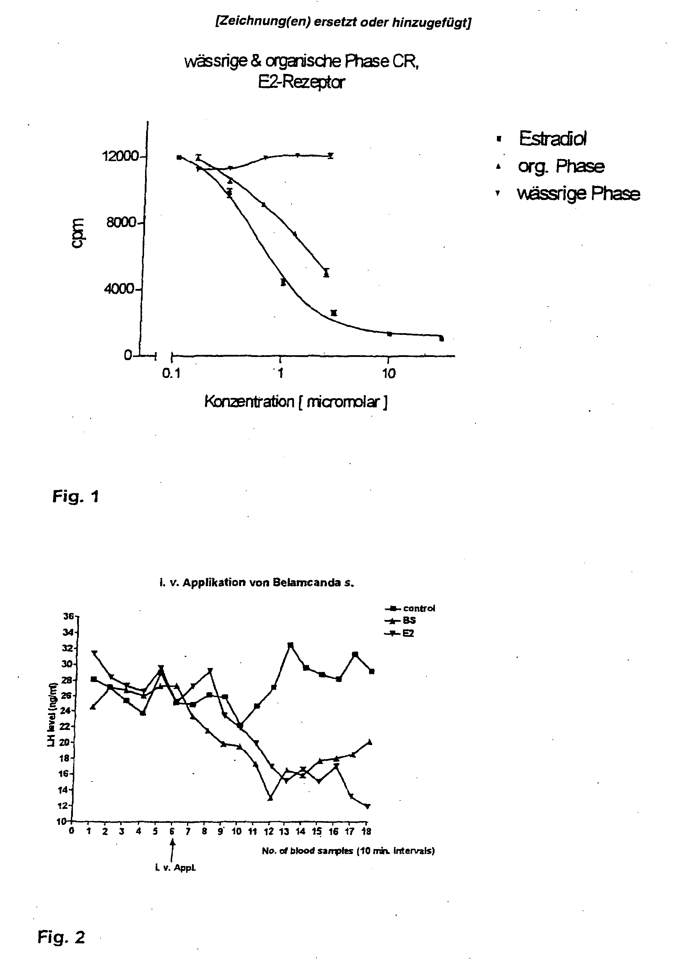

Die Ergebnisse sind in Fig. 1 gezeigt.

### 1.2 In vitro Belamcanda chinensis

Es ist aus anderen Untersuchungen bekannt, dass Extrakte aus Belamcanda chinensis ebenfalls Inhaltsstoffe besitzen, die von einem Antikörper gegen 17-β-Estradiol erkannt werden und an den 17ß-Estradiolrezeptor binden (vgl. Nenninger ibd.). Überraschenderweise haben die Erfinder der vorliegenden Anmeldung jedoch gefunden, dass diese Extrakte auf verschiedene Organsysteme unterschiedlich estrogen wirken, insbesondere, daß sie nicht uterotrop wirken.

### 2. in vivo Versuche: Nachweis der estrogenen Wirkung an der ovariektomierten Ratte

Die Bindung an den Rezeptor E2 ist sehr selektiv; es kann aber nicht ausgesagt werden, ob die nachgeschalteten Prozesse in der Zelle gefördert oder gehemmt werden, d.h. ob die Substanz ein Agonist oder ein Antagonist ist. Diese Eigenschaft kann nur in geeigneten zellulären Systemen oder im Ganztier bestimmt werden.

Die ovariektomierte Ratte ist anerkanntes Modell für die postmenopausale Frau, bei der die endogene Estradiol-Produktion zum Erliegen gekommen ist. Durch die externe Zufuhr von 17β-Estradiol oder von Stoffen, die estrogenartig wirken, kommt es zu einer Restauration estrogensensibler anatomisch-morphologischer Parameter wie Zunahme des Uterusgewichtes und dem Auftreten von verhornten d.h. Schollen-Epithelzellen am Vaginalepithel oder hormoneller Veränderungen, wie einem Absinken des LH-Spiegels im Blut der behandelten Tiere.
Alle im nachfolgenden beschriebenen Versuche wurden mit ovariektomierten Sprague-Dawley-Ratten (=ovx Ratten) mit einem Gewicht zwischen 240 und 280 g durchgeführt.

### 2.1 Einmalige Gabe von Belamcanda chinensis

Der Wirkungseintritt der Estradiol-artigen Wirkung von Belamcanda chinensis Extrakt erfolgt sehr rasch. Bereits nach einmaliger i.v. Gabe von Vehikel, Estradiol und Belamcanda chinensis Extrakt an ovx-Ratten sistiert die Pulsatilität unter E₂ wie auch unter Beiamcanda chinensis. Im Mittelwertverlauf ergeben sich signifikante Hemmungen der Serum-LH-Spiegel, sowohl im Vergleich zu den Vorwerten als auch im Vergleich zu den Cremophor-behandelten Kontrolltieren. Cremophor ist ein Emulgator auf Basis polyethoxylierter Ricinusölderivate.

Die Ergebnisse sind in Fig. 2 dargestellt.

Im Uterus der Tiere ist sechs Stunden nach Injektion des Belamcanda chinensis Extraktes die Expression der uterinen VEGF-, IGF1- und C3-Gene im Vergleich zu den Kontrollen nicht verändert, während die Estradiolinjektion eine deutliche Erhöhung der Genexpression dieser drej Estrogen-regulierten Proteine zur Folge hat. Das konstitutiv exprimierte CCO-Gen war durch keine der Behandlungen signifikant beeinflußt.

Aus den Befunden ergibt sich, dass Inhaltsstoffe von Belamcanda chinensis in hypothalamischen estrogen-rezeptiven Strukturen eine Hemmung des GnRH-Pulsgenerators bewirken und somit estrogen-agonistische Wirkungen haben. Dadurch wird die hypophysäre LH-Sekretion sowohl durch Inhaltsstoffe in Belamcanda chinensis als auch durch Estradiol signifikant gehemmt. Im Gegensatz zu Estradiol haben die Inhaltsstoffe in Belamcanda chinensis keine uterotrope Wirkung. Estradiol reguliert die Genexpression von VEGF, IGF1 und C3 signifikant herauf, ein Effekt, der unter Belamcanda chinensis nicht beobachtet wird.

Durchführung des Akutversuches zur Wirkung einer i.v.-Injektion von Belamcanda chinensis Extrakt
24 Ratten (i.e. 8 Tiere/Gruppe) wurden am Vortag des Versuches unter Äthernarkose Jugularvenenkatheter implantiert. Am Versuchstag wurden 6 Blutproben im Abstand von 10 Min. entnommen. Unmittelbar nach Entnahme der 6. Probenentnahme wurden 62,5 mg des Belamcanda chinensis Extraktes oder 10 µg 17β-Estradiol (E₂) bzw. das Lösungsmittel (5 %) Cremophor in isotoner NaCl 1 ml) intravenös injiziert und Blutproben für weitere 2 Stunden in 10minütigen Intervallen entnommen. 6 Stunden nach der intravenösen Gabe wurden die Tiere dekapitiert, Blut gewonnen und die Uteri entnommen, gewogen und in flüssigem Stickstoff tiefgefroren.

### 2.2 Einmalige Gabe von Tectorigenin

Nach einmaliger Gabe von Tectorigenin wurden der zeitliche Verlauf der Beeinflussung der LH-Spiegel im Blut und die Estradiol-ähnliche Immunreaktivität bestimmt. Die Konzentration von Tectorigenin im Blut der Tiere, bestimmt mittels E2-R1A, entspricht nach 20 min etwa 100 pg Äquivalent Estradiol.

Tectorigenin löst eine rasche Senkung des LH aus. Dabei entspricht die Kinetik der unter Tectorigenin erreichten LH-Senkung bis zum Zeitpunkt 60 min nach i.v. Gabe exakt derjenigen des Estradiols, führt dann aber zu keiner weiteren Senkung, sondern steigt langsam wieder an.

Durchführung: Ovx-Ratten wurden in Ethernarkose 24 Stunden vor Versuchsbeginn Katheter in die Vena jugularis externa nach der Methode von Harms und Ojeda gelegt (Harms PG; Ojeda SR: A rapid and simple procedure for chronic cannulation of the rat jugular vein. J. Appl. Physiol. (1974) 36: 391-392). Das Schlauchende wurde in eine Hauttasche im Nacken verlegt. Um die Tiere zur Gewinnung der Blutproben nicht berühren zu müssen, wurde der Katheter mit einem Silikonschlauch verlängert. Katheter und Schlauch wurden mit Ringerlösung, die 50 IU Heparin/ml enthielt, gespült.
Bei den Tieren wurde in 10 min Abständen Blutproben von 100 µl gezogen und das entnommene Volumen durch Ringer/Heparin-Lösung ersetzt. Nach der 6. Probe wurden 1,0 ml der jeweiligen Testlösung intravenös appliziert. Als Testlösungen wurden eingesetzt: 2% Cremophor (=Vehikellösung), Tectorigenin 7mg/ml Vehikel, 17β-Estradiol 10µg/ml Vehikel. Die Blutentnahme erfolgte in zehnminütigem Abstand über weitere 140 min.

Die so gewonnenen Blutproben wurden in ein 0,5 ml Eppendorf-Reaktionsgefäß, das 10 µl Heparin-Lösung (5000 IU/ml, Liquemin) enthielt gefüllt, 10 min bei 10 000 * g zentrifugiert und das Plasma bis zur Durchführung der Radioimmunoassays bei -20°C gelagert.
Die RIAs für LH und Prolaktin basieren auf Antiseren, Referenz- und Jodierungspräparaten des NIH (Bethesda, Maryland, USA). Die Konzentrationen von Estradiol und der kreuzreagierenden Isoflavone wurde mit einem RIA der Fa. DPC, Bad Nauheim gemessen.

### 2.3 Wirkung von Belamcanda chinensis Extrakt nach Verabreichung über 7 Tage

Die Auswirkungen der wiederholten Gabe von Estradiol, Belamcanda chinensis Extrakt und Vehikel auf Gesamtgewicht, Uterusgewicht, Hormonspiegel und Genaktivierung von Uterus und Knochen wurde an ovariektomierten Ratten nach täglicher s.c. Applikation über sieben Tage untersucht.

Die durchschnittlichen Körpergewichte der Cremophor- und Belamcanda chinensis behandelten Tiere unterscheiden sich nicht, während die E₂behandelten Tiere signifikant leichter waren. Auch die Uterusgewichte, der mit Cremophor- und Belamcanda chinensis behandelten Tieren unterscheiden sich nicht signifikant, während die E₂-Behandlung die Uterusgewichte mehr als verdreifachte.
Die Serum-LH-Spiegel bei den Belamcanda chinensis behandelten Tieren waren geringfügig, aber signifikant gegenüber den Cremophor-Kontrollen reduziert; deutlicher war die Reduktion durch Estradiol.

In dem uterinem mRNA-Extrakt erhöhte Estradiol nach einwöchiger Behandlung die Genexpression von VEGF signifikant auf 149% des Kontrollwertes. Unter Belamcanda chinensis Extrakt war die Expression leicht, aber nicht signifikant erhöht. Die Expression des nicht estrogen-regulierten konstitutiven Gens für die Cytochrom C Oxidase (= CCO) wurde nicht beeinflußt
In Extrakten des Femurkopfes wurde die Collagen-1A1-, die Osteocalcin-, die IGF1- sowie die TGFβ-mRNA-Expression bestimmt. Estradiol wie auch Belamcanda chinensis inhibierten die Expression aller 4 Gene signifikant, ohne einen Einfluß auf das konstitutive CCO-Gen auszuüben.

Die unterschiedliche Wirkung von Estradiol und Belamcanda kommt nach der siebentägigen Behandlung sehr deutlich zum Ausdruck. Belamcanda chinensis Extrakt hat auf die hypophysäre LH-Sekretion durch Hemmung des GnRH-Pulsgenerators sowie auf die Genexpression von vier estrogen-regulierten Genen im Knochen einen Estradiol-agonistischen Einfluß. Dagegen besteht keine estrogene Wirkung auf den Uterus: weder das Uterusgewicht noch das Estrogen-regulierte VEGF-Gen werden durch den Belamcanda chinensis Extrakt beeinflußt. Im Gegensatz dazu führt Estradiol zu einer Ballonierung des Uterus und zu einer Aktivierung des VEGF-Gens.

Durchführung des subakuten Versuches zur Wirkung von täglicher s.c.-Injektion über 7 Tage:
Jeweils 8 Tiere pro Versuchsgruppe (insgesamt 24) wurden täglich zwischen 08.00 und 09.00 Uhr 62,5 mg Belamcanda chinensis Extrakt bzw. 10 µg Estradiol oder dem Lösungsmittel (5%iges Cremophor, 1 ml) subkutan injiziert. 6 Std. nach der letzten Applikation wurden die Tiere dekapitiert und jedem Tier die Aorta, der Uterus und der linke Femurkopf entnommen, gesäubert und in flüssigem Stickstoff eingefroren.
In den Blutproben wurde LH und die Estradiol-Immunoreaktivität bestimmt.

### 2.4 Wiederholte Gabe von Cimicifuga racemosa

Die Tiere erhalten frühestens 14 Tage nach der Ovariektomie die jeweilige Prüfsubstanz in einer Dosis von 62,5 mg Cimicifuga racemosa/Ratte oder 8 µg Estradiol/Ratte einmal täglich morgens über einen Zeitraum von 7 Tagen subkutan injiziert. Beide Substanzen waren in 5% Cremophor gelöst, die Kontrolltiere erhielten nur das Vehikel.

Nach der Dekapitierung der Tiere wurden Gehirn, Uterus und Femur für die mRNA-Gewinnung präpariert. Im Blut der Tiere wurde mittels RIA die LH-Konzentration bestimmt. Die Expression der estrogen-regulierten Gene in den oben genannten Organen wurde mittels semiquantitativer RT-PCR bestimmt.

Die Uteri der Estradiol-behandelten Tiere sind mehr als dreimal so schwer wie die der Cimicifuga racemosa und Vehikel-behandelten Tiere, die sich in ihren Mittelwerten praktisch nicht unterscheiden. Das bedeutet, dass die Inhaltsstoffe von Cimicifuga racemosa keinen Einfluß auf die Gebärmutter der Tiere haben. Dies gilt auch für die Vagina, wo bei den Cimicifuga racemosa und Vehikel-behandelten Tiere keine Verhornung des Epithelgewebes auftritt, ganz im Gegensatz zu den Estradiol-behandelten Tieren.

Die LH-Spiegel der Vehikel-behandelten Tiere bleiben hoch, während sie sowohl durch Estradiol wie auch durch Cimicifuga racemosa signifikant gesenkt werden.

Die Ergebnisse sind in den Fig. 3a) und 3b) gezeigt.

| **Uterusgewichte (feucht)** | | | |
|---|---|---|---|
| | Cremophor [Kontrolle] | Cimicifuga racemosa | E2 |
| Anzahl Tiere | 8 | 8 | 8 |
| Mittelwerte [mg] | 185,6 | 192,3 | 702,1 |
| SD | 18,81 | 22,53 | 194,97 |
| SEM | 6,65 | 7,97 | 68,92 |

| **LH-Konzentrationen im Blut** | | | |
|---|---|---|---|
| | Cremophor [Kontrolle] | Cimicifuga racemosa | E2 |
| Anzahl Tiere | 8 | 8 | 8 |
| Mittelwerte [ng/ml] | 16,9 | 12,5 | 7,83 |
| SD | 3,99 | 3,4 | 5,57 |
| SEM | 1,41 | 1,2 | 1,97 |

Als weiterer Marker für die estrogene Wirkung wurde die Aktivierung von mRNA von estrogen-induzierbaren Proteinen gemessen. Untersucht wurde dabei Gewebe aus Uterus, aus Knochengewebe (Femur) und aus der präoptischen Region des Hypothalamus und.

Im Hypothalamus stimulieren sowohl Cimicifuga racemosa als auch E2 die Expression der mRNA für den Estrogenrezeptor α **(****Fig 4a****).** Auch im Knochengewebe verhält sich Cimicifuga racemosa wie ein Estrogen und verringert analog zu Estradiol die Expression der mRNA für die knochenspezifischen Kollagen 1 und für Osteocalcin-Gene **(****Fig 4b****)**.
Im Gegensatz dazu wird keine Wirkung von Cimicifuga racemosa auf estrogen-regulierte Gene im Uterus beobachtet. Nur Estradiol erhöht die mRNA für IGF1 und Komplementfaktor C3 **(****Fig 4c****).**

Diese Befunde belegen, daß die Inhaltsstoffe aus Cimicifuga racemosa selektiv auf einzelne Organe wirken: Der Extrakt wirkt estrogen im Hypothalamus ( Expression des E2-Rezeptors α, Freisetzung von LH) und am Knochen, nachgewiesen durch die Expression der Gene für Kollagen 1 und Osteocalcin. Im Gegensatz zu Estradiol besitzt Cimicifuga racemosa aber keine Wirkung auf den Uterus, wie die fehlende Wirkung auf die Uterusgewichte und die Expression der Gene für IGF1 und C3 zeigt.

Mit den in vitro und in vivo durchgeführten Experimenten konnte gezeigt werden, dass Cimicifuga racemosa- und Belamcanda chinensis - Extrakte eine estrogene Wirkung ausüben. Überraschend wurde gefunden, dass die Extrakte aus den genannten Drogen organselektiv an ZNS, Knochen und Gefäßen, aber nicht am Uterus wirken und sich damit hervorragend für die Prophylaxe und Therapie des Estrogenmangels eignen, ohne einen negativen Einfluss auf das Endometrium auszuüben.

Identische Effekte werden durch das in Belamcanda enthaltene Tectorigenin erzielt.

Somit stehen erstmals Arzneimittel zur Verfügung, die eine estrogenartige Wirkung, jedoch ohne uterotrope Wirkung aufweisen.

Derartige Arzneimittel gemäß Anspruch 1 und 4 können zur Behandlung und/oder Prophylaxe von kardiovaskulären Erkrankungen, insbesondere Atherosklerose, Osteoporose eingesetzt werden.

Derartige Arzneimittel gemäß Anspruch 5 können zur Behandlung und/oder Prophylaxe der Atherosklerose und Osteoporose eingesetzt werden.

Derartige Arzneimittel gemäß Anspruch 3 können zur Behandlung und/oder Prophylaxe der Atherosklerose eingesetzt werden.

Als Applikationsarten stehen die orale, intravenöse und subkutane Applikation im Vordergrund.

## Patentansprüche

1. Verwendung von Extrakten aus Irisgewächsen (Iridaceae) zur Herstellung eines östrogenartigen organselektiven Arzneimittels ohne oder wenigstens mit zu vernachlässigender uterotroper Wirkung zur Behandlung und/oder Prophylaxe von estrogenmangelbedingten Zuständen ausgewählt aus kardiovaskulären Erkrankungen, insbesondere Atherosklerose, und Osteoporose.

2. Verwendung nach Anspruch 1, **dadurch gekennzeichnet, dass** die Extrakte aus Belamcanda chinensis hergestellt werden.

3. Verwendung von Extrakten aus Cimicifuga racemosa zur Herstellung eines östrogenartigen organselektiven Arzneimittels ohne oder wenigstens mit zu vernachlässigender uterotroper Wirkung zur Behandlung und/oder Prophylaxe von estrogenmangelbedingter Atherosklerose; mit der Maßgabe, dass das Arzneimittel nicht zur Linderung von peri- und postmenopausalen Beschwerden sowie Dysmenorrhö verwendet wird.

4. Verwendung von Tectorigenin- und/oder Tectorigeninglykosid-haltigen Extrakten oder Tectorigenin- und/oder Tectorigeninglykosidangereicherten Extrakten zur Herstellung eines östrogenartigen organselektiven Arzneimittels ohne oder wenigstens mit zu vernachlässigender uterotroper Wirkung zur Behandlung und/oder Prophylaxe von estrogenmangelbedingten Zuständen ausgewählt aus kardiovaskulären Erkrankungen, insbesondere Atherosklerose, und Osteoporose.

5. Verwendung von Tectorigenin und/oder dessen Glykosiden zur Herstellung eines östrogenartigen organselektiven Arzneimittels ohne oder wenigstens mit zu vernachlässigender uterotroper Wirkung zur Behandlung und/oder Prophylaxe von Estrogenmangelzuständen ausgewählt aus Atherosklerose, Osteoporose.

## Claims

1. Use of extracts from iris plants (*Iridaceae*) for producing an estrogen-type, organoselective medicament having no uterotrophic effect or one that is at least negligible, for the treatment and/or prophylaxis of conditions brought about by estrogen deficiency selected from cardiovascular diseases, particularly atherosclerosis, and osteoporosis.

2. Use in accordance with claim 1, **characterised in that** the extracts are produced from *Belamcanda chinensis.*

3. Use of extracts from *Cimicifuga racemosa* for producing an estrogen-type, organoselective medicament having no uterotrophic effect or one that is at least negligible, for the treatment and/or prophylaxis of atherosclerosis brought about by estrogen deficiency; under the proviso that the medicament is not used for alleviating peri-menopausal and post-menopausal disorders and dysmenorrhea.

4. Use of extracts containing tectorigenin and/or tectorigenin glycoside or extracts enriched with tectorigenin and/or tectorigenin glycoside, for producing an estrogen-type, organoselective medicament having no uterotrophic effect or one that is at least negligible, for the treatment and/or prophylaxis of conditions brought about by estrogen deficiency selected from cardiovascular diseases, particularly atherosclerosis, and osteoporosis.

5. Use of tectorigenin and/or its glycosides for producing an estrogen-type, organoselective medicament having no uterotrophic effect or one that is at least negligible, for the treatment and/or prophylaxis of conditions brought about by estrogen deficiency selected from atherosclerosis, osteoporosis.

## Revendications

1. Utilisation d'extraits d'iris (iridacées) pour la fabrication d'un médicament organosélectif du type oestrogène, sans, ou au moins avec une action utérotrope négligeable, pour le traitement et/ou la prophylaxie des états **caractérisés par** un manque d'oestrogènes choisis parmi des maladies cardiovasculaires, en particulier l'athérosclérose et l'ostéoporose.

2. Utilisation selon la revendication 1, **caractérisée en ce que** les extraits sont fabriqués à partir de *Belamcanda chinensis.*

3. Utilisation d'extraits de *Cimicifuga racemosa* pour la fabrication d'un médicament organosélectif du type oestrogène, sans, ou au moins avec une action utérotrope négligeable, pour le traitement et/ou la prophylaxie de l'athérosclérose **caractérisée par** un manque d'oestrogènes ; à condition que le médicament ne soit pas utilisé pour soulager les troubles péri- et post-ménauposiques, ni les dysménorrhées.

4. Utilisation d'extraits contenant de la tectorigénine et/ou du glycoside de tectorigénine ou des extraits enrichis en tectorigénine et/ou en glycoside de tectorigénine pour la fabrication d'un médicament organosélectif du type oestrogène sans ou au moins avec une action utérotrope négligeable pour le traitement et/ou la prophylaxie des états **caractérisés par** un manque d'oestrogènes choisis parmi des maladies cardiovasculaires, en particulier l'athérosclérose et l'ostéoporose.

5. Utilisation de tectorigénine et/ou de ses glycosides pour la fabrication d'un médicament organosélectif du type oestrogène sans ou au moins avec une action utérotrope négligeable pour le traitement et/ou la prophylaxie des états **caractérisés par** un manque d'oestrogènes choisis parmi l'athérosclérose et l'ostéoporose.
